# NEW EUROPEAN PATENT SPECIFICATION

(11) **EP 0 093 599 B2**
(45) Date of publication and mention of the opposition decision: **30.08.1995**
(45) Mention of the grant of the patent: 10.02.1988
(21) Application number: 83302443.3
(22) Date of filing: 29.04.1983
(51) Int. Cl.: A61B 1/07, G02B 23/26

(54) **Hard endoscope of the oblique viewing type**
Nicht beugsames Endoskop mit schiefwinkliger Sichtrichtung
Endoscope rigide du type à visualisation oblique

(30) Priority: 01.05.1982 JP 74239/82
(43) Date of publication of application: 09.11.1983
(73) Proprietor: OLYMPUS OPTICAL CO., LTD., Tokyo 151 (JP)
(72) Inventor: Hashiguchi, Toshihiko, Sagamihara-shi Kanagawa-ken (JP); Ikegami, Yoshie, Ina-shi Nagano-ken (JP); Isono, Kesao, Ina-shi Nagano-ken (JP)
(74) Representative: Bridge-Butler, Alan James

(56) References cited:
- EP-A- 0 700 652
- US-A- 3 297 022
- US-A- 3 896 793
- US-A- 4 072 147
- Katalog Urologie der Fa. Storz, pp. 681-6, 1979
- Katalog "UROSET" der Karl Storz KG mit dem Druckvermerk 10-68
- Katalog "UROLOGY" der Karl Storz KG mit dem Druckvermerk 671
- Instruction Manual "Urology" der Karl Storz KG mit dem Druckvermerk 1271
- Artikel "A new optical system ..." von Dr. G. Berci et al.
- Katalogblatt der Fa. Storz-Surgical Instruments Copyright 1992
- Endoscopy by George Berci, Appleton-Century-Crafts, New York, 1976

## Description

The present invention relates to endoscopes of the type having an outer housing that is substantially rigid, hereinafter referred to as "hard endoscopes", to distinguish them from endoscopes of a pliant, flexible construction. Furthermore, the endoscopes to which the invention relates are of the "oblique viewing" type, which have a distal end to the housing which is inclined to the longitudinal axis thereof, to give an oblique field of view in a forward direction inclined to the axis. The endoscopes with which the invention is concerned are provided with means to illuminate the field of view.

In recent years, endoscopes have become widely employed for inspection and diagnosis of intracavitary organs, or the wallsurface of body cavities. In the industrial field, such endoscopes may be employed to inspect the interior of boiler tubes, machines, chemical plants, etc.

In such endoscopes incorporating an illuminating optical system for projecting illuminating light from the endoscope tip, the light is transmitted from a source at the observer's end, to be projected from the output port when inserted in a cavity, and a viewing optical system must be provided for inspecting the field of view via an eye-piece at the observer's end, the image being transmitted by said viewing optical system, after being focussed on the illuminated object via a viewing aperture adjacent the output port.

In order to be able to inspect objects appropriately according to the positional requirements for observing a desired region through said viewing optical system when illuminated from the light source, endoscopes may be designed for viewing in the axial direction in front of a tubular insertion sheath fed in through an opening, the illuminating light beams projected accordingly, and such endoscopes are classified as being of the direct view type. Endoscopes may be designed to give a lateral view, from the side of the tubular insertion sheath. In the case of an endoscope designed to give an oblique view, inclined at a predetermined angle with respect to the longitudinal axis of the tubular insertion sheath, there are particular problems in obtaining an even illumination of the field of view.

In hard endoscopes of oblique viewing type, made using current techniques, an inner optical system tube passes within an outer insertion sheath containing the illumination-transmitting means in a cavity of substantially crescent-shaped cross-section formed between the inner circumference of the outer insertion-sheath tube and the outer circumference of said inner tube, so that the object illuminated by light projected from the tip surface of said transmission means may be inspected via the viewing optical system. However, as in the endoscope of oblique viewing type disclosed in our European Patent Application 0 070 652, of Japanese priority, the illuminating light projected from the tip of a light guide fibre bunch which forms a transmission means in the insertion sheath, the light projected in the oblique direction is diffused, and cannot evenly illuminate the required field of view. Accordingly, even if the object is well placed in the field of view, it is often difficult to inspect the details thereof sufficiently clearly in the reproduced image, and therefore diagnosis is impeded. Furthermore, it is difficult to maintain a given direction of illumination with high accuracy during the production of a quantity of endoscopes.

One object of the present invention is to provide a hard endoscope of the oblique viewing type which evenly-illuminates the required field of view, but concentrates the illumination, and enables a tighter tolerance to be maintained during production of a quantity of such endoscopes.

According to the present invention there is provided a hard endoscope having a rigid tubular housing forming an insertion sheath extending from an operational hand-grip that is provided with a light-guide entry port and an eyepiece and terminating in a planar end-face at the distal end, the normal to the end face being inclined to the longitudinal axis of the endoscope, in which end face a viewing aperture is provided adjacent a single output-port for illumination projected from an end-face of a single light-guide fibre bunch, an optical viewing system being provided in the sheath and extending adjacent the insertion sheath between the eyepiece and the viewing aperture and sealed at the end-face by an end-face member defining the viewing aperture and also defining a generally crescent-shaped aperture for the fibre bunch, characterised in that the light-guide fibres in the bunch adjacent the output port are trapped between two opposed part-cylindrical surfaces so as to maintain the fibres in the bunch substantially parallel in order to limit the dispersion of the projected light.

The invention will now be described with reference to the drawings, in which:-
Figure 1 is a cross-section showing the front end of one known construction of a hard endoscope of the oblique viewing type by way of example;
Figure 2 is a schematic side view of one exemplary embodiment of a hard endoscope of the oblique viewing type constructed in accordance with the invention;
Figure 3(a) is a longitudinal sectional view of the front end of the tubular housing of the embodiment shown in Figure 2;
Figure 3(b) is a perspective, part-sectional view of the embodiment shown in Figure 2;
Figure 4 is a longitudinal section showing a machined workpiece for use in forming the outer insertion sheath of the embodiment shown in Figure 2;
Figure 5(a) is an explanatory diagram showing details of a working process for forming the tubular, optical system housing member as a longitudinal part-sectional view;
Figure 5(b) is a front view showing details of the housing member tip;
Figure 6 is a longitudinal section showing the tip of a second exemplary embodiment of the present invention;
Figure 7 is a top view showing the tip of a third exemplary embodiment of the present invention;
Figure 8 is a perspective view of a tool for use in constructing the third exemplary embodiment of the present invention;
Figure 9 is a top view showing the tip of a fourth exemplary embodiment of the present invention;
Figure 10 is a top view showing the tip of a fifth exemplary embodiment of the present invention;
Figure 11(a) is a longitudinal sectional view of the tip of a sixth exemplary embodiment of the present invention; and
Figure 11(b) is a top view of the embodiment shown in Figure 11(a).

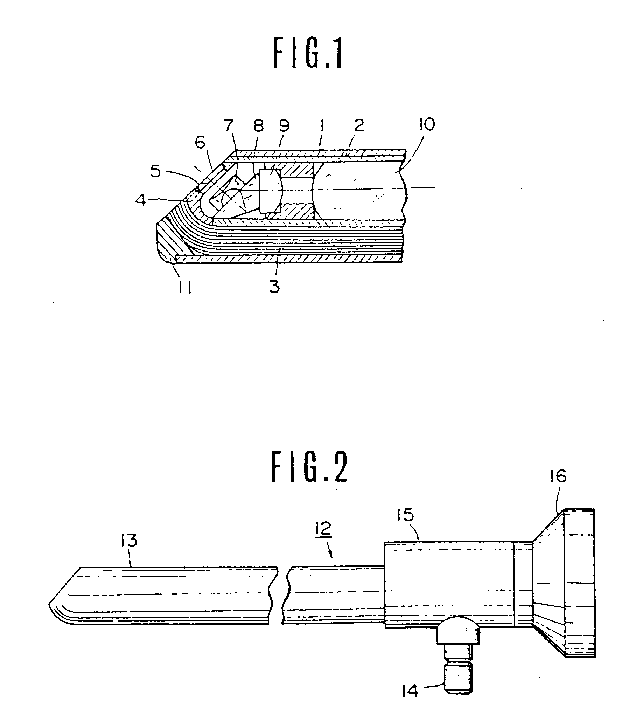

The known hard endoscope of the oblique viewing type shown in Figure 1 comprises an inner tube 2 containing the optical viewing system of the endoscope, which tube is arranged eccentrically inside an outer tube 1 forming an extended insertion sheath which also contains a light-guide fibre bunch 3 for transmitting light from a source (not shown), to be projected from the tip, the fibre bunch 3 being positioned in the substantially crescent-shaped cavity formed between the outer periphery of the inner tube 2 and the inner surface of the tube 1.

An oblique opening normal to the viewing direction is formed at the tip of the outer tube 1, and a support member 4 defines a viewing aperture mounted at the tip of the inner tube 2.

The viewing aperture in the support member 4 is fitted with a cover glass 6 secured by a frame 5. In the space sealed within said cover glass 6, an optical image of the object positioned in the field of view inclined to the longitudinal axis of the tubular housing is focussed to the rear in the axial direction of the tubular housing via an objective optical system formed by a front lens 7, prism 8 and rear lens 9, and the image is relayed onto the eyepiece system through a relay lens system 10.

The tip of the light-guide fibre bunch 3 arranged about the periphery of the inner tube 2 is guided and curved along the inner periphery of a tip member 11 mounted between the outer periphery of the support member 4 and the tip of the outer tube 1, so as to form an output port to illuminate an object from a zone outside the outer periphery of the cover glass 6, in an oblique direction.

In this known hard endoscope of the oblique viewing type, since the curvature of the outer peripheral surface of the support member 4 is different from the curvature of the inner surface of the tip member 11, the light-guide fibre bunch 3 has fibres that are not arranged mutually parallel in the output port. Therefore, the illuminating light projected from the output port at the tip of the light-guide fibre bunch 3 tends to be dispersed, and some light pases out of the field of view of the optical system and is lost, while the visible region is not evenly illuminated. Accordingly, the object region to be inspected is difficult to observe clearly, and it is difficult to assure high accuracy with regard to the direction of the central path of the projected illumination when producing a quantity of endoscopes. Embodiments of the present invention are designed to avoid these difficulties found in the known endoscope constructions.

In a first exemplary embodiment, shown in Figure 2, a hard endoscope of the oblique viewing type is composed of an extended tubular insertion sheath 13, an operational hand-grip section 15 with a light-guide entry port 14 extending from one side at the rear end of the sheath 13, and an eyepiece 16 containing an eyepiece optical system, not represented in detail at the rear of the operational hand grip section 15.

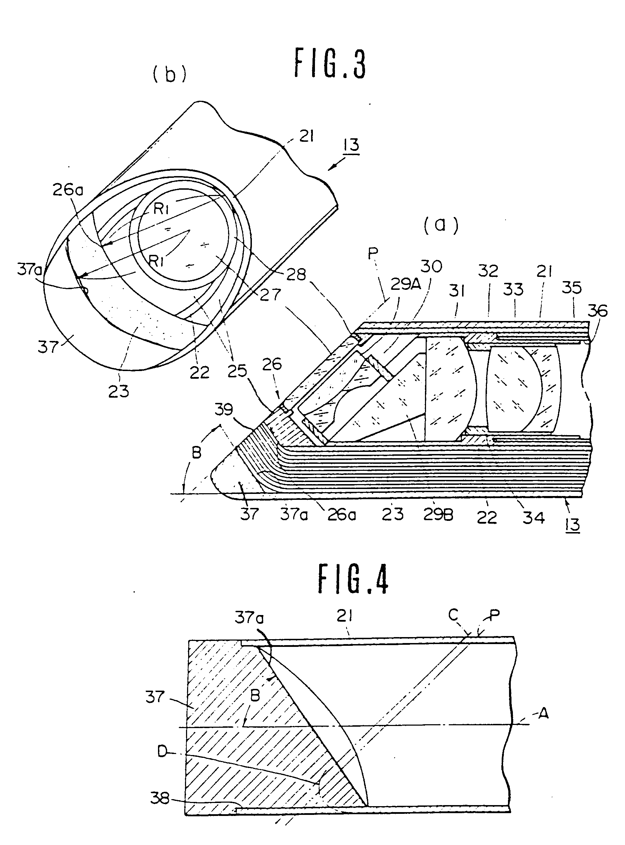

As can be seen in Figure 3, the tubular insertion sheath 13 is formed by a rigid outer tube 21, in which an inner tube 22 containing the optical viewing system is arranged eccentrically, in contact with the upper inner surface of the outer tube 21. In the outer tube 21, about the outer periphery of the inner tube 22, a light-guide fibre bunch 23 is provided for transmitting illumination supplied to said light-guide entry-port 14 to be projected from the tip face.

The tip of said outer tube 21 is cut on a line normal to the direction of the field of view, and forms an open surface inclined with a predetermined angle to the longitudinal axis of the tubular housing 12. The tip of the inner tube 22, arranged eccentrically inside this opening, is bored to provide a viewing aperture. An end face member 25 on the inner tube has its outer rim attached to the inner wall of said opening to form a substantially tubular housing member 26 enclosing the optical viewing system. A cover glass 27, with its partial outer periphery cut as a taper, is fixed to the viewing aperture in the end face member 25 of the inner tube by pushing a fastening ring 28 into the opening to press the tapered peripheral edge of the cover glass 27 to make the peripheral edge of the other surface butt onto the tapered peripheral edge.

An objective optical system to be described below is contained inside the viewing aperture sealed by the cover glass 27. A front lens 29A is arranged parallel to the plane of the cover glass 27, with a slight gap, and is fixed to a prism 29B by a frame 30.

An objective lens 31 is fitted to the back plane of the prism 29B, arranged normal to the longitudinal axis of the housing. The periphery of the lens 31 is fixed to an objective support frame 32. A rear objective lens system 33 is separated from the objective lens 31 by a predetermined distance, and is fixed to the objective support frame 32 by an intermediate frame 34.

The objective support frame 32 is fitted to the inner tube 22. The rear end of the objective support frame 32 is diametrically reduced stepwise, and fitted to be connected rearwardly to the inner surface of a tubular container 35 containing a relay optical system (not shown) by an intermediate ring spacer 36.

The tubular member of the present invention is composed in such manner that a tip member 37 of the external tube is attached at the forward tip of the external tube 21, at its outer periphery, separated from the member 26 incorporating the end face member 25 of the inner tube 22 to form a sealed closure.

The tip of the outer tube 21 and its tip member 37 are formed as shown in Figure 4, for example. A tip member 37 with a cylindrical fitting section 38 having an outer diameter substantially mating with the inner diameter of the outer tube 21 is securely connected to the end of the outer tube 21, for example by a soldered joint in the case of metal tubes, and preferably using a silver solder.

The inner end face of the tip member 37 of the outer tube is formed with a part-cylindrical surface 37a of a radius R₁ inclined at an angle B (as shown in Figure 3) relative to the longitudinal axis A of the outer tube 21. The tubular housing is cut away at an angle substantially normal to said part-cylindrical surface 37a to form an end face C that can be subjected to inspection before polishing and finishing. This tip member 37 of an outer tube is only exposed at the lateral lower part (as drawn) projecting forwards on the end plane C. This part projected with a sharp angle of the tip end member 37 now has its front edge rounded, as shown by a chain-dotted line D, to avoid any danger of injury to patients at the time of introducing the endoscope into a body cavity.

The end plane C cutaway as described above, then has the light guide fibre bunch 23 fitted via the tube 21,so that the end of the fibre bunch is in close alignment with the part-cylindrical face 37a, and the fibres secured in position, for example by an assembly tool. The end-face is then polished till the part shown as a chain-dotted line P forms the end face plane 39 for oblique illumination and viewing, as shown in Figure 3. In this way, the outer tube 21 is incorporated with a tip member 37 of predetermined shape, and the inner tubular member is so formed to enter with its member 26 and the light-guide fibre bunch 23. In this case, the inner periphery of the part-cylindrical internal end surface of the tip member 37 for said outer tube becomes a part-cylindrical surface 37a having a radius of curvature R₁, as shown in Figure 3.

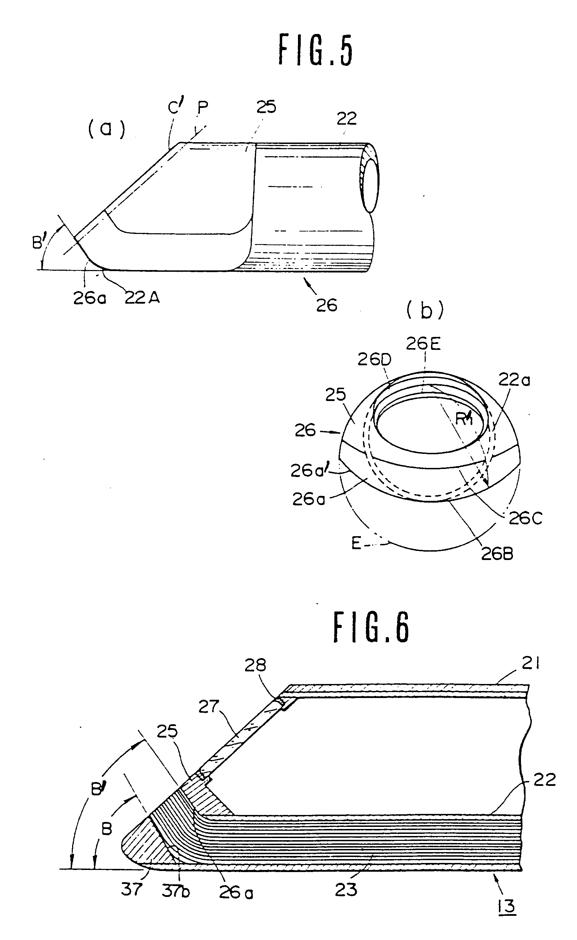

On the other hand, the end face member 25 is incorporated with the inner tube 22 to form a closure member 26 of substantially tubular shape as shown in Figure 5.

In Figure 5a, the end of the inner tube 22 is fixed to the rear end of an insertion sheath part having an outer diameter equal to the inner diameter of the outer tube 21 when considered in the direction normal to the plane of the drawing as shown by the chain-dotted line E in Figure 5(b), and the front side of this cylindrical member is cut away on an inclined plane C' (see Figure 5(a)) while leaving a margin for abrasive polishing (thicker portion), and the outer front face of this cylindrical member is cut to give a peripheral section with a radius of curvature R₁ substantially about the central axis of the part-cylindrical surface, or the direction of the field of view to form a part-cylindrical surface 26a on the lower front side (as drawn) with an angle B' (equal to the angle B in this case) with respect to the longitudinal axis of the inner tube 22. Furthermore, a part-cylindrical surface 26' having a peripheral section with a radius of curvature R' and with its central axis parallel with the central axis of the inner tube 22 is formed in contact with the lower part of the inner tube 22 by machining. In this case, reference numbers 26B and 26C in Figure 5(b) indicate the respective boundaries of these part-cylindrical surfaces 26a' and 26a. A cylindrical viewing aperture is formed in the-upper portion on the inclined plane C'. When the inclined plane C' is ground away to reduce the members to a predetermined thickness by polishing, and after the housing member 26 has been passed eccentrically through the outer tube 21, the embodiment having the structure shown in Figure 3 is obtained.

In Figure 5(b), a concave facing 26D is shown in which is pushed a ring fastener 28 for the cover glass 27, and a flange 26E projected inwards from said concave facing 26D is shown on the inner peripheral surface in the viewing aperture. Furthermore, the end 22a of the inner tube 22 is exposed as a ring in the inclined plane C' and in the part-cylindrical surface 26a. The common boundary of the part-cylindrical surface 26a of the end face member 25 for the inner tube, incorporated with the end of the inner tube 22, and the outer periphery of the inner tube 22 exposed at the outer periphery of said cylindrical surface 26a is rounded as shown at 22A in Figure 5(a).

The end face member 25 projected on the outer periphery of said inner tube 22, leaves the outer peripheral part on the upper side of the inner tube 22 (as shown) with a smaller wall thickness, while both sides of the outer peripheral parts have a larger wall thickness. The gap with a substantially crescent-shaped profile in cross section, remaining between the inner peripheral surface of the outer tube 21 and the outer peripheral surface of the inner tube 22 is closed on both sides of the inner tube to the outer periphery in the upper half near the tip, but the lower side of the end face member 25 of the inner tube is rounded at the distal side. The light-guide fibre bunch 23 passing through the substantially crescent-shaped gap within the inner tube 22 from the observer's side are collected together at the lower side of the inner tube 22 by the end face member 25 blocking both the sides of the inner tube 22 and the outer periphery at the upper side near the top. In short, the light-guide fibre bunch 23, near their output port end surfaces, pass through the gap in such manner that the cross sectional area formed between the part-cylindrical surface 26a, the outer peripheral surface on the lower side of the end face member 25 of the inner tube, and the part-cylindrical surface 37a, the inner peripheral surface of the tip member 37 of the outer tube, may be constant.

In this way the first exemplary embodiment has an end face member 25 mounted at the tip, with the member 26 containing the optical viewing system so adapted that its peripheral surface along the field of view may be a part-cylindrical surface 26a. Also the tip member 37 mounted at the end of the outer tube 21 has the part-cylindrical surface 37a substantially equally distanced from the part-cylindrical surface 26a. Therefore the projection surface of the light-guide fibre bunch 23 filling the output port that is defined between the part-cylindrical surfaces 26a and 37a are so adapted that the fibres lie mutually parallel along the direction of the field of view. That is why the illumination projected from the end surface of the fibre bunch 23 is directed positively in the field of view, and does not disperse much, so that it evenly and effectively illuminates an object visible in the field of view, enabling a clearly focussed image to be formed by the viewing optical system.

In the first described exemplary embodiment, the output port for the projection of light from the light-guide fibre bunch is defined by two part-cylindrical surfaces, 26a and 37a, and the width of its cross-section is constant, in spite of any dispersion of the fibres during processing, so that the number of light-guide fibres across the gap is substantially constant at any point along its length. It is advantageous, therefore, that inadequate or dispersed illumination is avoided, and the centre of the output illumination is reproducible in a stable manner when producing a quantity of endoscopes.

The angles, B and B' between each part-cylindrical surface, 26a and 37a, and the longitudinal axis of the outer tube 21, are both equal to or larger than the angle between the field of view and the longitudinal axis, in the embodiment described. Accordingly, in cases where these angles are equal to the angle of the field of view, the centre of the field of view will be well within the illumination, for any object relatively distant from the tip of the tubular housing. By making the angles larger than the angle of the field of view, the direction of the field of view will intersect the illumination at a position nearer to the tip of the tubular housing, and an excellent quality of illumination is obtained at the position of intersection. Therefore, an excellent illumination can be obtained in different cases, by selectively using endoscopes whose angles B, B' are selected appropriately, depending on whether an object to be inspected is distant or near.

A second exemplary embodiment is shown in Figure 6, in which the inclination of the angle B of the part-cylindrical surface 37b for the tip member 37 of the outer tube, with respect to the longitudinal axis of the tubular housing 13 is slightly larger than the inclination angle B' of the part-cylindrical surface 27a of the end face member of the inner tube comprising the member 26. Alternatively, the inclination angle B' of the part-cylindrical surface 26a may be made smaller than the inclination angle B, with equal effect.

In this exemplary embodiment, the cross-sectonal area of the output port containing the light-guide fibre bunch 23 reduces as the end is approached. As the filling level of the fibres grows higher, during polishing the end faces a wider gap is formed, and this helps to ensure that filled adhesive agent is not abrased too much, and that the top face does not become rough.

In this exemplary embodiment, the other details are the same as described for the embodiment shown in Figures 2 and 3, and the same elements have the same reference numbers. In Figure 6, the optical viewing system is not shown, for the sake of simplicity.

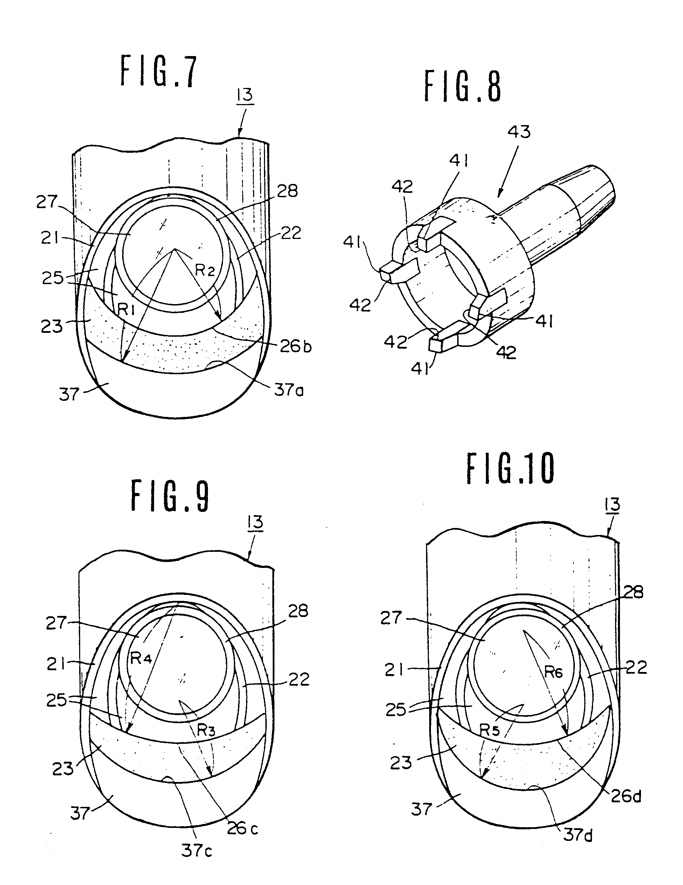

Figure 7 shows a third exemplary embodiment, in which the radius of curvature R₂ of the lower periphery (as drawn) of the part-cylindrical surface 26b of the end face member 25 of the inner tube is smaller than the radius of curvature R₁ of the periphery of the part-cylindrical surface 37a of the tip member 37 of the outer tube, and both part-cylindrical surfaces, 26b and 37a, have the same centre.

Accordingly, a tool 43 for shaping both part-cylindrical surfaces 26b, 37a, can be as shown in Figure 8, for example, with plural cutting elements each having a cutting edge 41 for machining an internal wall to a radius of curvature R₁ and a cutting edge 42 for machining an outer peripheral surface to a radius of curvature R₂, the elements being mounted at the end of a cylindrical tool body. If the cutting-tool 43 can cut the material coming in contact with the cutting edge by rotation, both part-cylindrical surfaces 26b and 37a can be machined with a single tool 43 to effect a cost reduction.

In a fourth exemplary embodiment, shown in Figure 9, the radius of curvature R₃ of a part-cylindrical surface 37c of the tip member 37 on the outer tube is smaller than the radius of curvature R₄, of a peripheral section of the part-cylindrical surface 26c on the end face member 25 of the inner tube.

In other words, the profile of the output port defined between the two part-cylindrical surfaces, 26c, 37c, wherein the light guide fibre bunch 23 passes through to the projection end face, is substantially crescent-shaped in cross-section, and it resembles the cross-section of the light-guide fibre bunch 23 which is passed through from the rear end of the tubular insertion sheath housing 13. Therefore when the fibre bunch 23 is inserted, the cross-sectional shape of the part that is to be curved changes slightly, longitudinally, and this so facilitates assembly that it is advantageously simple.

Figure 10 shows a fifth exemplary embodiment, in which the radius of curvature R₅ of a peripheral section of the part-cylindrical surface 37d of the tip member 37 on the outer tube is one half of the internal diameter of the outer tube 21, and the radius of curvature R₆ of the peripheral section of a part-cylindrical surface 26d of the end face member 25 on the inner tube is equal to the radius of the curvature R₁ of the peripheral section of a part-cylindrical surface 26a at the bottom surface of the end of the inner tube, shown in Figure 5.

With this construction, the cross-sectional profile of the curved part in the output port does not change, to facilitate inspection of the light-guide fibre bunch 23.

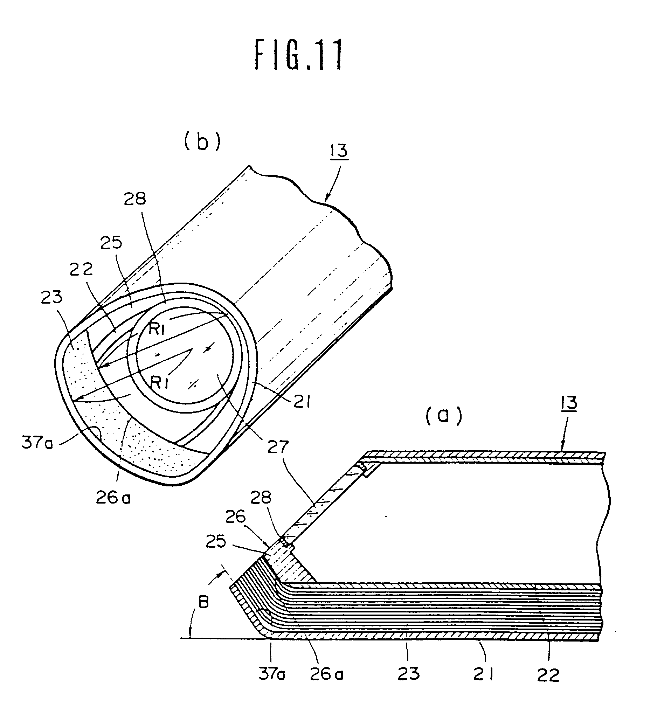

In a sixth exemplary embodiment, shown in Figures 11a and 11b, the production process comprises inserting the front end of the outer tube 21 into a mold under pressure, without employing a tip member 37 for the outer tube, and so molding a curved, part-cylindrical surface 37a.

With such a process, there is no connection part shown in Figure 4, which has to be prepared by soldering or otherwise securing in place, so that the user can employ the endoscope without risk of any such tip member 37 becoming detached and left in a body cavity. Otherwise, the details of this sixth embodiment may be described for any other embodiment described above, and the optical system is not shown in Figure 11, purely for the sake of simplicity.

The means for molding a curved tip on the outer tube 21, without employing a separate tip member 37 is obviously applicable to the other embodiments described.

In the above description, both the outer peripheral surface of the end of member 26 and the tip member 37 of outer tube or the inner peripheral surface of the end of the outer tube 21 are expressed as part-cylindrical surfaces. The use of a relay lens system for an image transmitting means is not essential, as a light guide may be incorporated in the optical system, for example, and the optical system can be modified in many ways to meet particular requirements.

Furthermore, the term "outer insertion sheath" as used in the following claims, is not intended to limit the protection to embodiments in which the tubular housing containing both the optical system in its own tube, and also an optical fibre bunch is the outermost tube of the endoscope construction.

## Claims

1. A hard endoscope having a rigid tubular housing forming an insertion sheath (13) extending from an operational hand-grip (15) that is provided with a light guide entry port (14) and an eyepiece (16) and terminating in a planar end-face at the distal end, the normal to the end face being inclined to the longitudinal axis of the endoscope, in which end face a viewing aperture (27) is provided adjacent a single output-port for illumination projected from an end-face (39) of a single light-guide fibre bunch (23) an optical viewing system being provided in the insertion sheath and extending adjacent the insertion sheath between the eyepiece (16) and the viewing aperture (27) and sealed at the end-face (39) by an end-face member (25) defining the viewing aperture and also defining a generally crescent-shaped aperture for the fibre bunch (23), characterised in that the light guide fibres in the bunch adjacent the output port are trapped between two opposed part-cylindrical surfaces (26,37) so as to maintain the fibres in the bunch substantially parallel in order to limit the dispersion of the projected light.

2. A hard endoscope as claimed in Claim 1, characterised in that are two opposed part-cylindrical surfaces (26a, 37a) defining the periphery of the output port, having substantially the same radius of curvature (R₁) (Fig. 3).

3. A hard endoscope as claimed in Claim 2, characterised in that the two peripheral surfaces (26b, 37a) are concentrically arranged (Fig. 7).

4. A hard endoscope as claimed in Claim 3, characterised in that the radius of curvature (R₂) of the innermost part-cylindrical surface (26b) is smaller than the radius of curvature (R₁) of the part-cylindrical surface (37a) forming part of said insertion sheath.

5. A hard endoscope as claimed in Claim 1, characterised in that said peripheral surfaces are both substantially part-cylindrical surfaces, and the radius of curvature (R₅) of the part-cylindrical surface (37d) in said insertion sheath (21) is one half of the internal diameter of said insertion sheath, and less than the radius of curvature (R₈) of the part-cylindrical surface (26d) on the tubular optical viewing system (Fig. 10).

6. A hard endoscope as claimed in Claim 1, characterised in that the two peripheral surfaces are both substantially part-cylindrical surfaces, and the angles of inclination (B) formed between each cylindrical surface and the longitudinal axis of the insertion sheath are equal.

7. A hard endoscope as claimed in Claim 1, characterised in that the two peripheral surfaces are both substantially part-cylindrical surfaces and the angle of inclination of the part-cylindrical surface (37b) on the sealing member (25) for the tubular optical viewing system (22) with respect to the longitudinal axis of the insertion sheath is larger than the angle of inclination (B') of the part-cylindrical surface (26a) formed on the insertion sheath with respect to that axis (Fig. 6).

8. A hard endoscope as claimed in Claim 1, characterised in that the part-cylindrical surface (37a) in said insertion sheath is formed by pressing the end of the insertion sheath to deform its tip (Fig. 11).

9. A hard endoscope as claimed in Claim 8, characterised in that the two peripheral surfaces are both substantially part-cylindrical surfaces, and the angle of inclination (B) formed by the part-cylindrical surface (37a) on said insertion sheath with respect to the longitudinal axis of the insertion sheath is equal to the angle of inclination between the direction of the field of view and the longitudinal axis of the insertion sheath (Fig. 11).

10. A hard endoscope as claimed in Claim 2, characterised in that the two peripheral surfaces are both substantially part-cylindrical surfaces, and the angle of inclination of the cylindrical surface on said insertion sheath with respect to the longitudinal axis of the insertion sheath is larger than the angle of inclination of the field of view with respect to said axis.

## Patentansprüche

1. Hartes Endoskop mit einem starren, rohrförmigen Gehäuse, das die Einführungshülle (13) bildet, die sich vom Bedienungshandgriff (15), der mit einem Lichtführungseinlaß (14) und einem Augenteil (16) versehen ist bis zu einer planaren Stirnfläche am distalen Ende erstreckt, wobei die Normale auf der Stirnfläche gegenüber der Endoskoplängsachse geneigt ist und wobei die Stirnfläche eine Sichtöffnung (27) nahe einem einzigen Auslaß für die Beleuchtung aufweist, die von einer Stirnfläche (39) eines einzigen Lichtleitfaserbündels (23) erfolgt und wobei ferner ein optisches Betrachtungssystem in der Einführungshülle vorgesehen ist, das sich nahe der Einführungshülle zwischen dem Augenteil (16) und der Sichtöffnung (27) befindet und an der Stirnfläche (39) durch ein Endteil (25) verschlossen ist, das die Sichtöffnung und allgemein die mondförmige Öffnung für das Faserbündel (23) definiert, **dadurch gekennzeichnet**, daß die Lichtleitfasern in dem Bündel nahe der Auslaßöffnung zwischen zwei gegenüberliegenden teilzylindrischen Oberflächen (26, 27) eingeschlossen sind, um so die Fasern in dem Bündel im wesentlichen parallel zu halten und die Dispersion des projizierten Lichts zu begrenzen.

2. Hartes Endoskop nach Anspruch 1, **dadurch gekennzeichnet**, daß zwei gegenüberliegende teilzylindrische Oberflächen (26a, 37a) vorgesehen sind, die die Auslaßöffnung definieren und die im wesentlichen denselben Kurvenradius (R₁) (Fig. 3) aufweisen.

3. Hartes Endoskop nach Anspruch 2, **dadurch gekennzeichnet**, daß zwei periphere Oberflächen (26b, 37a) konzentrisch angeordnet (Fig. 7) vorgesehen sind.

4. Hartes Endoskop nach Anspruch 3, **dadurch gekennzeichnet**, daß der Kurvenradius (R₂) der inneren teilzylindrischen Oberfläche (26b) kleiner ist als der Kurvenradius (R₁) der teilzylindrischen Oberfläche (37a), die einen Teil der Einführungshülle bildet.

5. Hartes Endoskop nach Anspruch 1, **dadurch gekennzeichnet**, daß die peripheren Oberflächen beide im wesentlichen teilzylindrische Oberflächen sind und daß der Kurvenradius (R₅) der teilzylindrischen Oberfläche (37d) in der Einführungshülle (21) die Hälfte des inneren Durchmessers der Einführungshülle beträgt und geringer ist als der Kurvenradius (R₈) der teilzylindrischen Oberfläche (26d) des rohrförmigen Betrachtungssystems (Fig. 10).

6. Hartes Endoskop nach Anspruch 1, **dadurch gekennzeichnet**, daß die zwei peripheren Oberflächen beide im wesentlichen teilzylindrische Oberflächen sind und daß die Neigungswinkel (B) zwischen jeder zylindrischen Oberfläche und der Längsachse der Einführungshülle gleich sind.

7. Hartes Endoskop nach Anspruch 1, **dadurch gekennzeichnet**, daß die zwei peripheren Oberflächen beide im wesentlichen teilzylindrische Oberflächen sind und der Neigungswinkel der teilzylindrischen Oberfläche (37b) auf dem Dichtungsteil (25) für das rohrförmige optische Betrachtungssystem (22) mit Bezug auf die Längsachse der Einführungshülle größer ist als der Neigungswinkel (B') der teilzylindrischen Oberfläche (26a) auf der Einführungshülle mit Bezug auf diese Achse (Fig. 6).

8. Hartes Endoskop nach Anspruch 1, **dadurch gekennzeichnet**, daß die teilzylindrischen Oberfläche (37a) in der Einführungshülle durch Pressen des Endes der Hülle zur Deformation der Spitze (Fig. 11) gebildet ist.

9. Hartes Endoskop nach Anspruch 8, **dadurch gekennzeichnet**, daß die zwei peripheren Oberflächen beide im wesentlichen teilzylindrische Oberflächen sind und daß der Neigungswinkel (B), der durch teilzylindrische Oberflächen (37a) auf der Einführungshülle mit Bezug auf die Längsachse der Hülle gleich dem Neigungswinkel zwischen der Richtung des Betrachtungsfeldes und der Längsachse der Einführungshülle ist (Fig. 11).

10. Hartes Endoskop nach Anspruch 2, **dadurch gekennzeichnet**, daß die zwei peripheren Oberflächen beide im wesentlichen teilzylindrische Oberflächen sind und daß der Neigungswinkel der zylindrischen Oberfläche auf der Einführungshülle mit Bezug auf die Längsachse der Hülle größer ist als der Neigungswinkel des Betrachtungsfeldes mit Bezug auf diese Achse.

## Revendications

1. Endoscope rigide comprenant un logement tubulaire rigide formant une gaine d'insertion (13) s'étendant depuis une poignée de manoeuvre (15) munie d'un orifice d'entrée formant un guide de lumière (14) et d'un oculaire (16) et se terminant par une face d'extrémité plane à l'extrémité distale, la normale à la face d'extrémité étant inclinée par rapport à l'axe longitudinal de l'endoscope, une ouverture d'observation (27) étant prévue dans ladite face d'extrémité, dans une position adjacente à un orifice de sortie destiné à l'éclairement projeté à partir d'une face d'extrémité (39) d'un seul faisceau de libres (23) formant guide de lumière, un système optique étant prévu dans la gaine d'insertion et s'étendant dans une position adjacente à la gaine d'insertion entre l'oculaire (16) et l'orifice d'observation (27) et fixé de façon étanche à la face d'extrémité (39) par un organe de face d'extrémité (25) définissant l'orifice d'observation et définissant également un orifice en forme générale de croissant pour le faisceau de fibres (23), caractérisé en ce que les libres conductrices de lumière du faisceau qui sont adjacentes à l'orifice de sortie sont maintenues captives entre deux surfaces partiellement cylindriques et opposées (26, 37) de manière à maintenir les fibres du faisceau sensiblement parallèles en vue de limiter la dispersion de la lumière projetée.

2. Endoscope rigide selon la revendication 1, caractérisé en ce que sont prévues deux surfaces partiellement cylindriques et opposées (26a, 37a) définissant la périphérie de l'orifice de sortie, et présentant sensiblement le même rayon de courbure (R₁) (Figure 3).

3. Endoscope rigide selon la revendication 2, caractérisé en ce que les deux surfaces périphériques (26b, 37a) sont disposées de façon concentrique (Figure 7).

4. Endoscope rigide selon la revendication 3, caractérisé en ce que le rayon de courbure (R₂) de la surface partiellement cylindrique le plus à l'intérieur (26b) est plus petit que le rayon de courbure (R₁) de la surface partiellement cylindrique (37a) faisant partie de ladite gaine d'insertion.

5. Endoscope rigide selon la revendication 1, caractérisé en ce que lesdites surfaces périphériques sont toutes les deux des surfaces sensiblement cylindriques, et le rayon de courbure (R₅) de la surface partiellement cylindrique (37d) dans ladite gaine d'insertion (21) est la moitié du diamètre interne de ladite gaine d'insertion, et inférieur au rayon de courbure (R₆) de la surface partiellement cylindrique (26d) du système optique tubulaire (Figure 10).

6. Endoscope rigide selon la revendication 1, caractérisé en ce que les deux surfaces périphériques sont toutes les deux des surfaces partiellement cylindriques, et les angles d'inclinaison (B) formés entre chaque surface cylindrique et l'axe longitudinal de la gaine d'insertion sont égaux.

7. Endoscope rigide selon la revendication 1, caractérisé en ce que les deux surfaces périphériques sont toutes les deux des surfaces sensiblement partiellement cylindriques et l'angle d'inclinaison de la surface partiellement cylindrique (37b) de l'organe de fixation étanche (25) destiné au système optique tubulaire (22) par rapport à l'axe longitudinal de la gaine d'insertion est plus important que l'angle d'inclinaison (B') de la surface partiellement cylindrique (26a) formée sur la gaine d'insertion par rapport à cet axe (Figure 6).

8. Endoscope rigide selon la revendication 1, caractérisé en ce que la surface partiellement cylindrique (37a) de ladite gaine d'insertion est formée par compression de l'extrémité de la gaine d'insertion en vue de déformer sa pointe (Figure 11).

9. Endoscope rigide selon la revendication 8, caractérisé en ce que les deux surfaces périphériques sont toutes les deux des surfaces sensiblement partiellement cylindriques, et l'angle d'inclinaison (B) formé entre la surface partiellement cylindrique (37a) de ladite gaine d'insertion et l'axe longitudinal de la gaine d'insertion est égal à l'angle d'inclinaison entre la direction du champ d'observation et l'axe longitudinal de la gaine d'insertion (Figure 11).

10. Endoscope rigide selon la revendication 2, caractérisé en ce que les deux surfaces périphériques sont toutes les deux des surfaces sensiblement partiellement cylindriques, et l'angle d'inclinaison de la surface cylindrique de ladite gaine d'insertion par rapport à l'axe longitudinal de la gaine d'insertion est plus important que l'angle d'inclinaison du champ d'observation par rapport audit axe.
